# EUROPEAN PATENT SPECIFICATION

(11) **EP 4 205 681 B1**
(45) Date of publication and mention of the grant of the patent: **27.05.2026**
(21) Application number: 22217131.6
(22) Date of filing: 29.12.2022
(51) Int. Cl.: A61B 18/14, A61B 5/283

(54) **BASKET CATHETER HAVING ABLATION ELECTRODES AND ELECTRO-ANATOMICAL SENSING ELECTRODES**
KORBKATHETER MIT ABLATIONSELEKTRODEN UND ELEKTROANATOMISCHEN MESSELEKTRODEN
CATHÉTER À PANIER COMPORTANT DES ÉLECTRODES D'ABLATION ET DES ÉLECTRODES DE DÉTECTION ÉLECTRO-ANATOMIQUE

(30) Priority: 30.12.2021 US 202117566272
(43) Date of publication of application: 05.07.2023
(73) Proprietor: Biosense Webster (Israel) Ltd., Yokneam, 2066717 (IL)
(72) Inventor: GOVARI, Assaf, Yokneam 2066717 (IL); BEECKLER, Christopher Thomas, Irvine 92618 (US); KEYES, Joseph Thomas, Irvine 92618 (US); LICHTER, Justin George, Irvine 92618 (US); HERRERA, Kevin Justin, Irvine 92618 (US)
(74) Representative: Carpmaels & Ransford LLP

(56) References cited:
- WO-A1-2021/001772
- WO-A1-2021/126980
- US-A1- 2015 366 508
- US-A1- 2016 073 960
- US-A1- 2017 049 349
- US-A1- 2017 164 858

## Description

### FIELD OF THE DISCLOSURE

The present disclosure relates generally to medical devices, and particularly to production methods and expandable catheters having ablation electrodes and electro-anatomical sensing electrodes.

### BACKGROUND OF THE DISCLOSURE

Producing methods and expandable catheters having ablation electrodes and various types of sensing electrodes have been published.

For example, U.S. Patent Application Publication 2020/0337765 describes systems, devices and methods for modulating targeted nerve fibers (e.g., hepatic neuromodulation) or other tissue. The systems may be configured to access tortuous anatomy of or adjacent hepatic vasculature. The systems may be configured to target nerves surrounding (e.g., within adventitia of or within perivascular space of) an artery or other blood vessel, such as the common hepatic artery.

U.S. Patent Application Publication 2017/0049649 relates to flexible electrodes and methods for making flexible electrodes for use in a cardiac mapping catheter.

### SUMMARY OF THE INVENTION

The invention is defined in claims 1 and 9. Advantageous embodiments of the invention are defined in the dependent claims.

The present disclosure will be more fully understood from the following detailed description of the examples thereof, taken together with the drawings in which:

### BRIEF DESCRIPTION OF THE DRAWINGS

Fig. 1 is a schematic, pictorial illustration of a catheter-based position-tracking and ablation system, in accordance with an example of the present disclosure;
Fig. 2 is a schematic, pictorial illustration of a distal-end assembly of a basket catheter in an expanded position, in accordance with examples of the present disclosure;
Fig. 3 is a schematic top view of multiple sensing electrodes as produced, and a sectional view of one sensing electrode, in accordance with examples of the present disclosure;
Fig. 4 is a block diagram that schematically illustrates a process flow for producing one of the sensing electrodes shown in Figs 2 and 3, in accordance with examples of the present disclosure;
Fig. 5 is a flow chart that schematically illustrates a method for producing a basket catheter having ablation electrodes and the sensing electrodes shown in Figs. 2-4, in accordance with an example of the present disclosure; and
Fig. 6 is a flow chart that schematically illustrates a method for producing a basket catheter having ablation electrodes and one or more thermocouples, in accordance with an example of the present disclosure.

### DETAILED DESCRIPTION OF EXAMPLES

### OVERVIEW

Examples of the present disclosure that are described hereinbelow provide improved techniques for producing an expandable catheter, such as a basket catheter, having ablation electrodes, electro-anatomical (EA) sensing electrodes and temperature sensors, such as thermocouples (TCs).

In some examples, an ablation catheter comprises a shaft for insertion into a patient heart, and an expandable distal end, in the present example, a basket-shaped distal-end assembly, also referred to herein as a basket. The basket is coupled to the shaft and is configured to be in a collapsed position when moved through the vasculature to or from the heart, and to be expanded to one or more expanded positions, e.g., for sensing electrocardiogram (ECG) signals and/or for applying ablation signals to tissue of the heart.

The basket comprises multiple arms, also referred to herein as splines. In some examples, at least one of, and typically all the splines, comprise a flexible substrate, which is configured to conform to tissue of the heart when being placed in contact therewith, so as to: (i) sense ECG signals in the tissue, and/or (ii) apply radiofrequency (RF) ablation signals (e.g., pulses) to the tissue via the aforementioned ablation electrodes.

In some examples, at least one of and typically each ablation electrode includes a slot, which is configured to contain a temperature sensing device, such as a thermocouple (TC). When a given ablation electrode that contains a given TC is placed in contact with the tissue, the given TC is configured to produce a thermal signal indicative of the sensed temperature of the tissue. Note that the TC is configured to sense the tissue temperature while the ablation electrode is applying ablation signals to the tissue, and/or when the ablation electrodes is in contact with the tissue, but no ablation signal is applied to the tissue.

In some examples, when placed in contact with tissue of the heart, each of the EA sensing electrodes, also referred to herein as a sensing electrode for brevity, is configured to produce a electrically signal indicative of an ECG signal sensed in the tissue. Each spline may comprise one or more sensing electrodes that are coupled to the flexible substrate at selected positions along a longitudinal axis of the spline.

In some examples, each sensing electrode comprises a gold substrate whose surface includes at least first and second sections that do not overlap one another, but have a common interface.

In some examples, a first polymer layer, which is formed over the first section of the gold substrate, is configured to electrically isolate between the tissue and the gold substrate. A second polymer layer, which is formed over the second section of the gold substrate, is configured to conduct the ECG or electrical signal between the tissue and the gold substrate.

In some examples, a plurality of the sensing electrodes may be produced together and then separated into one or more sensing electrodes intended to be coupled to a respective spline.

In some examples, the gold substrate includes a first surface intended to be coupled to the flexible substrate of the spline, and a second surface having the first and second sections.

In some examples, in the production process flow: (i) the first polymer layer is applied to the entire surface of the gold substrate, (ii) the first polymer layer is removed from the second section (e.g., using a laser that cuts through the first polymer layer) for exposing the second surface of the gold substrate, and (iii) the second polymer layer is applied to the second section of the second surface of the gold substrate.

In some examples, the second outer surface of the second polymer is recessed relative to the first outer surface of the first polymer, so as to protect the second outer surface from being damaged when placed in contact with the tissue.

In other examples, the outer surface of the first and second polymer layers, which are intended to be placed in contact with the tissue, are typically flush with one another. For example, these examples are obtained by having a similar thickness of the first and second polymer layers.

In alternative examples, the outer surface of the second polymer layer may slightly protrude (e.g., about less than 5 µm) toward the tissue relative to the outer surface of the first polymer layer, so as to conduct the ECG signal from the tissue to the gold substrate by having sufficient contact force between the tissue and the outer surface of the second polymer layer. For example, these examples are obtained by having the thickness of the second polymer slightly layer is larger than that of the first polymer layer.

In some examples, at least one of and typically each spline of the basket catheter may have on their surface intended to be placed in contact with the tissue: (i) at first positions, the one or more ablation electrodes and temperature sensors contained within the slots of the ablation electrodes, and (ii) at second positions that are different from the first positions, the one or more sensing electrodes.

The disclosed techniques improve the functionality of expandable catheters, such as basket catheters, for conducting both electro-anatomical mapping and ablation of tissue in question. Moreover, the disclose techniques reduce the need to insert two different catheters (sensing and ablation) into the patient heart, and therefore, reduce the duration of the medical procedure and reduce the cost associated with using two different catheters for performing the sensing and ablation procedures.

### SYSTEM DESCRIPTION

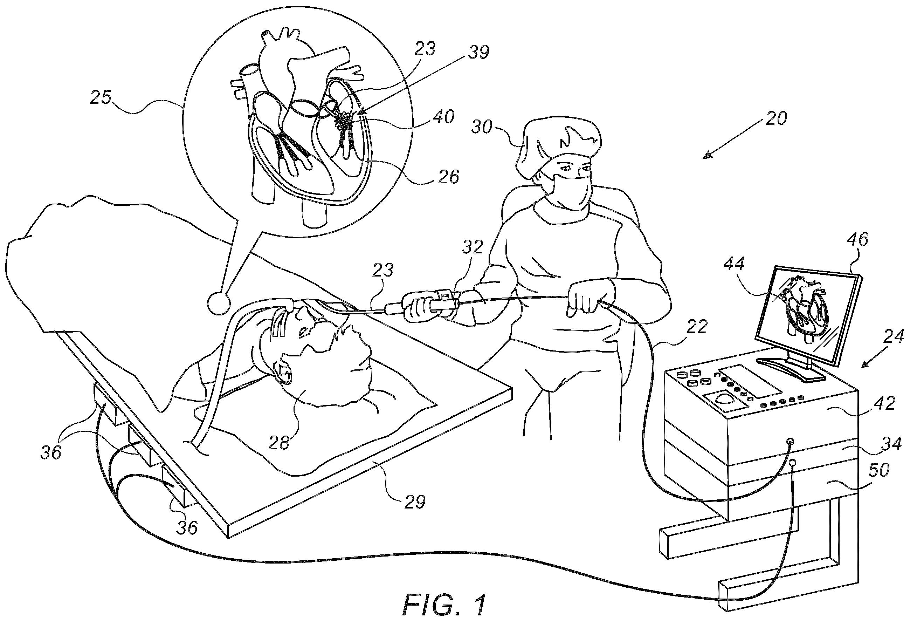

Fig. 1 is a schematic, pictorial illustration of a catheter-based position-tracking and ablation system 20, in accordance with an example of the present disclosure. In some examples, system 20 comprises a catheter 22, in the present example an expandable cardiac catheter having a basket shape, and a control console 24. In the example described herein, catheter 22 may be used for any suitable therapeutic and/or diagnostic purposes, such as but not limited to sensing of electro-anatomical (EA) information in tissue in question and applying ablation signals to tissue of a heart 26. In the context of the present disclosure, the term information refers to the spatial location of the catheter distal end, and an electrocardiogram (ECG) signal sensed by electrodes of catheter 22.

In some examples, console 24 comprises a processor 42, typically a general-purpose computer, with suitable front end and interface circuits for receiving signals from catheter 22 and for controlling other components of system 20 described herein. Processor 42 may be programmed in software to carry out the functions that are used by the system, and is configured to store data for the software in a memory 50. The software may be downloaded to console 24 in electronic form, over a network, for example, or it may be provided on non-transitory tangible media, such as optical, magnetic or electronic memory media. Alternatively, some or all of the functions of processor 42 may be carried out using an application-specific integrated circuit (ASIC) or any suitable type of programmable digital hardware components.

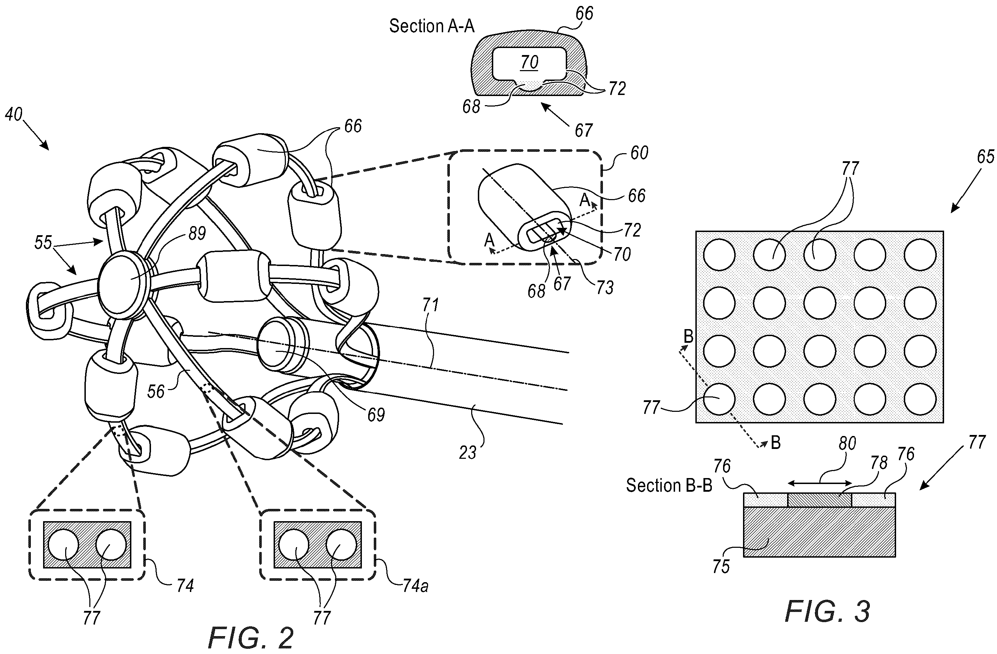

Reference is now made to an inset 25. In some examples, catheter 22 comprises a distal-end assembly 40 having multiple splines (shown in detail in Fig. 2 below), and a shaft 23 for inserting distal-end assembly 40 to a target location for ablating tissue in heart 26. During an Electrophysiology (EP) mapping and/or ablation procedure, physician 30 inserts catheter 22 through the vasculature system of a patient 28 lying on a table 29. Physician 30 moves distal-end assembly 40 to the target location in heart 26 using a manipulator 32 near a proximal end of catheter 22, which is connected to interface circuitry of processor 42.

In some examples, catheter 22 comprises a position sensor 39 of a position tracking system, which is coupled to the distal end of catheter 22, e.g., in close proximity to distal-end assembly 40. In the present example, position sensor 39 comprises a magnetic position sensor, but in other examples, any other suitable type of position sensor (e.g., other than magnetic-based) may be used.

Reference is now made back to the general view of Fig. 1. In some examples, during the navigation of distal-end assembly 40 in heart 26, processor 42 receives signals from magnetic position sensor 39 in response to magnetic fields from external field generators 36, for example, for the purpose of measuring the position of distal-end assembly 40 in heart 26. In some examples, console 24 comprises a driver circuit 34, configured to drive magnetic field generators 36. Magnetic field generators 36 are placed at known positions external to patient 28, e.g., below table 29.

In some examples, processor 42 is configured to display, e.g., on a display 46 of console 24, the tracked position of distal-end assembly 40 overlaid on an image 44 of heart 26.

The method of position sensing using external magnetic fields is implemented in various medical applications, for example, in the CARTO^{™} system, produced by Biosense Webster Inc. (Irvine, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

### DISTAL-END ASSEMBLY HAVING ABLATION ELECTRODES SENSING ELECTRODES AND TEMPERATURE SENSORS

Fig. 2 is a schematic, pictorial illustration of distal-end assembly 40 of catheter 22 in an expanded position, in accordance with examples of the present disclosure. In some examples, physician 30 may collapse and expand distal-end assembly 40 using manipulator 32, as will be described below.

In some examples, distal-end assembly 40 comprises an expandable basket catheter, which is coupled between shaft 23 and an apex 89 of distal-end assembly 40. Note that apex 89 is the distal-most part of catheter 22 which is typically (but not necessarily) orthogonal to an axis 71 of shaft 23.

In the present example, distal-end assembly 40 comprises multiple splines 55. Each spline 55 is made from or comprises a flexible substrate 56, such as an alloy of nickel-titanium (e.g., nitinol), or from any other one or more suitable materials, such as a multi-layered flexible printed circuit board. Note that splines 55 must be sufficiently flexible to conform to the tissue in question when being placed in contact therewith.

In some examples, distal-end assembly 40 comprises one or more ablation electrodes 66, in the present example, one or more ablation electrodes 66 are assembled on at least a given spline 55, and typically on each spline 55 of distal-end assembly 40.

Reference is now made back to an inset 60, showing one implementation of ablation electrode 66 in accordance with examples of the present disclosure.

In some examples, ablation electrode 66 includes a through hole (TH) 70 formed along an axis 73 of ablation electrode 66 and therethrough, so that spline 55 could be threaded through TH 70 as shown in the general view of Fig. 2.

In some examples, ablation electrode 66 includes a slot 67 which is formed in an inner surface 72 of TH 70, and is configured to contain a temperature sensor, such as a thermocouple (TC) 68, or any suitable device other than a temperature sensor. Slot 67 may be formed on any interior surface 72 of TH 70, and the preferred location of slot 67 is in the side of electrode 66 that is intended to face the tissue in question of heart 26.

Reference is now made to a sectional view AA of ablation electrode 66. In some examples, inner surface 72 is shaped such that TC 68 fits into and fills slot 67 without protruding into the area of TH 70, which is configured to snuggly fit over spline 55.

In some examples, when ablation electrode 66 is placed in contact with a given tissue of heart 26 that is intended to be ablated, TC 68 is configured to produce a thermal signal indicative of the temperature of the given tissue. In such examples, spline 55 is configured to: (i) conduct ablation signal(s) produced in console 24 and applied to the given tissue via ablation electrode 66, and (ii) conduct the thermal signal from TC 68 to processor 42 for analyzing the temperature of the given tissue. Note that TC 68 is controlled by processor 42 and is configured to produce the thermal signal: (i) while applying the ablation signal(s) to the given tissue, and (ii) when the ablation signal(s) are not applied to the given tissue.

Reference is now made back to the general view of Fig. 2. In some examples, when physician 30 moves distal-end assembly 40 of catheter 22 to the target location in heart 26, distal-end assembly 40 is in a collapsed position with all splines 55 straightened. When distal-end assembly 40 is positioned at the target location in heart 26, physician 30 typically reduces the distance between a distal end 69 of shaft 23 and apex 89 (e.g., by pulling apex 89 toward shaft 23, or by pushing distal end 69 of shaft 23 toward apex 89, or using any other technique), so as to have distal-end assembly 40 at an expanded position with splines 55 bent as shown in the example of Fig. 2. In the present example, physician 30 may use manipulator 32 (shown in Fig. 1 above) for controlling the distance between apex 89 and distal end 69 of shaft 23, and therefore, the amount of expansion of distal-end assembly 40. Alternatively, splines 55 may be shape set so that their natural shape is of a roughly spherical basket which is allowed to collapse when put into a sheath (not shown).

In some examples, distal-end assembly 40 comprises various types of diagnostic electrodes, such as sensing electrodes 77, which are coupled to (or formed on or disposed on) spline 55 and are typically positioned between adjacent ablation electrodes 66, or between an ablation electrode 66 and another element (e.g., apex 89 and/or shaft 23) coupled to spline 55. Note that at each position, spline 55 may have one or more sensing electrodes 77.

In some examples, at least one of and typically all sensing electrodes 77 are configured to be coupled to spline 66. When placed in contact with tissue of heart 26, each sensing electrode 77 is configured to produce a electrically signal indicative of an electrocardiogram (ECG) signal sensed from the tissue.

In some examples, processor 42 is configured to produce an EA map of the tissue in question (and other sections of heart 26). Based on the EA map, physician 30 (and/or processor 42) may determine one or more locations for applying the ablation signals to the tissue. Note that in principle it is possible to insert into heart 26 an additional catheter having sensing electrodes, such as sensing electrodes 77, for performing the EP mapping before or while inserting an ablation electrode. However, insertion of an additional catheter may: (i) prolong the ablation procedure, (ii) increase the risk for a safety event due to insertion of two catheters, and (iii) increase the cost associated with using two catheters instead of a single catheter having both sensing electrodes and ablation electrodes.

Reference is now made to insets 74 and 74a showing two pairs of sensing electrodes 77 coupled to each spline 55 at a selected position. In the example of inset 74 two sensing electrodes 77 are positioned between adjacent ablation electrodes 66, and in the example of inset 74a two sensing electrodes 77 are positioned between an ablation electrode 66 and apex 89.

In other examples, any other suitable number of sensing electrodes 77 may be implemented at each suitable location along spline 55. For example, one sensing electrode 77 between ablation electrode 66 and apex 89 and two or more sensing electrodes 77 implemented between two adjacent ablation electrodes 66.

In some examples, each sensing electrode 77 includes a circular shape and a diameter of about 0.15 mm. In alternative examples, the size and shape of each sensing electrode 77 may vary based on the position within each spline, or between splines. For example, a first sensing electrode 77 may have a square shape having a width of about 0.14 mm, a second sensing electrode 77 may have a circular shape having a diameter of about 0.15 mm, and a third sensing electrode 77 may have a circular shape having a diameter of about 0.18 mm.

As used herein, the terms "about" or "approximately" for any numerical values or ranges indicate a suitable dimensional tolerance that allows the part or collection of components to function for its intended purpose as described herein.

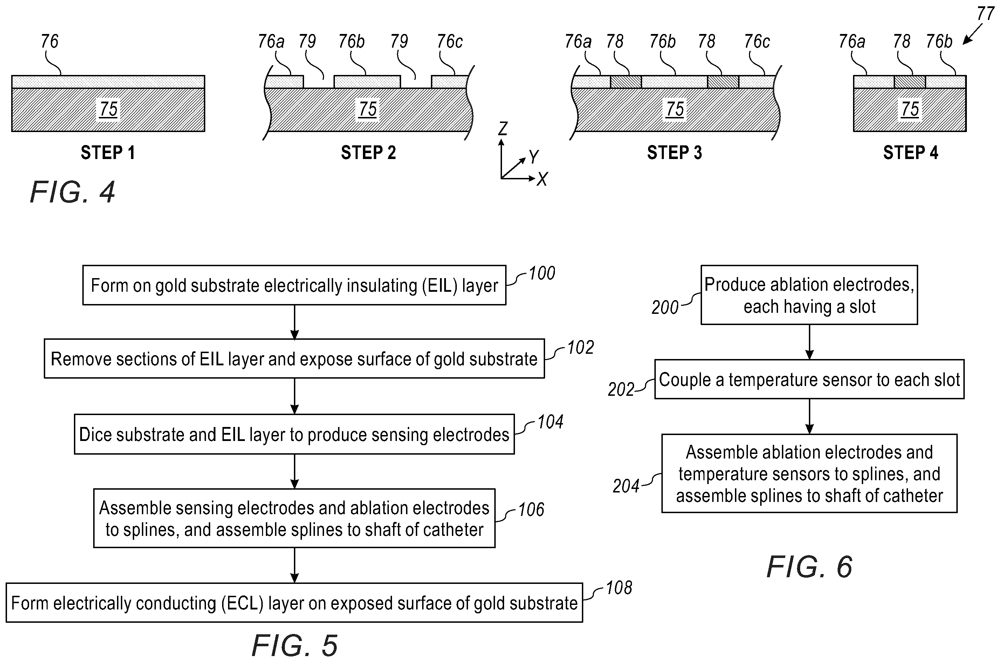

In some examples, each sensing electrode 77 includes an electrically conductive section and an electrically insulative section. Sensing electrodes 77 and their production processes are described in detail below in Figs. 3 and 4, respectively.

This particular configuration of distal-end assembly 40 is shown by way of example, in order to illustrate certain problems that are addressed by examples of the present disclosure and to demonstrate the application of these examples in enhancing the performance of such a catheterization and tissue sensing and ablation system. Examples of the present disclosure, however, are by no means limited to this specific sort of example distal end assembly, and the principles described herein may similarly be applied to other sorts of distal end assemblies and/or catheters used in tissue sensing, tissue ablation, a combination thereof and other medical applications applied to other organs of patient 28.

Note that by having ablation electrodes 66, TCs 68 and sensing electrodes 77 combined on distal-end assembly 40, physician 30 may insert distal-end assembly 40 into a cavity (e.g., right atrium or left atrium of heart 26), perform electro-anatomical mapping, and subsequently, perform the tissue ablation without retracting the catheter and/or inserting an additional catheter. In other words, the EA sensing, the temperature sensing, and the tissue ablation are carried out using one catheter having distal-end assembly 40 as shown in Fig. 2.

Additionally, or alternatively, sensing electrodes 77 may be implemented in distal end 40 using any other suitable technique. For example, using microelectrodes that are described in detail in U.S. Patent Applications 16/723,971 and 17/489,895 (which is a continuation-in-part of U.S. Patent Application 16/723,971). In some examples, distal-end assembly 40 may comprise these microelectrodes together with ablation electrodes 66 and TCs 68.

Fig. 3 is a schematic top view of multiple sensing electrodes 77 as produced, and a sectional view BB of one sensing electrode 77, in accordance with examples of the present disclosure.

In some examples, multiple sensing electrodes 77 are produced in one batch, e.g., in an array 65. Subsequently, sensing electrodes 77 are diced and separated into a single sensing sensor 77 or into a smaller array of multiple (e.g., a pair of) sensing sensors 77, as shown for example, in insets 74 and 74a of Fig. 2 above. The production process of sensing electrode(s) 77 is designed to reduce the manufacturing cost and is described in more detail in Fig. 4 below.

Reference is now made to sectional view BB of a selected sensing electrode 77. In some examples, each sensing electrode 77 comprises a gold substrate 75, which is formed on flexible substrate 56 of spline 55 and is configured to conduct the electrically signal(s) produced by the respective sensing electrode 77.

In some examples, sensing electrode 77 comprises a first polymer layer 76, which is formed over a first section of gold substrate 75 and is configured to electrically isolate between the tissue in question and gold substrate 75. In some examples, first polymer layer 76 may comprise an electrically insulating polymer, such as but not limited to polyethylene terephthalate (PET) or polyether block amide (PEBA).

In some examples, sensing electrode 77 comprises a second polymer layer 78, which is formed over a second section of gold substrate 75, and is configured to conduct the electrical signal between the tissue in question and gold substrate 75. Second polymer layer 78 is positioned between two sections of first polymer layer 76, i.e., the second section includes a position different than that of the first section.

In some examples, second polymer layer 78 comprises an electrically conductive polymer, such as but not limited to poly(3,4-ethylenedioxythiophene) (PEDOT), poly(3, 4 ethylenedioxythiophene) polystyrene sulfonate (PEDOT:PSS), or any other suitable type of electrically-conductive polymer. In other examples, layer 78 may comprise non-polymer materials, such as electrochemically grown iridium oxide, electrochemically grown Titanium Nitride (TiN) or any other suitable type of electrically conductive alloy. In some examples, layer 78 is configured to reduce the impedance of electrode 77, and includes a width 80, in the present example, a diameter of about 0.15 mm, as described in Fig. 2 above.

In some examples, the outer surface of polymer layer 78 is recessed relative to the outer surface of polymer layer 76. In such examples, when placed in contact with tissue, the outer surface of polymer layer 76 prevents direct contact with the interior of a sheath but allows for contact with the tissue, so as to prevent damage, such as erosion, to the surface of polymer layer 78.

In other examples, the outer surface of polymer layers 76 and 78 are flush with one another, so that when sensing electrode 77 is placed in contact with the tissue in question, both polymer layers 76 and 78 have a firmed contact with the tissue in question.

In alternative examples, the outer surface of polymer layer 78 slightly protrudes (e.g., about less than 5 µm) relative to the outer surface of polymer layer 76, so as to improve the sensing of the ECG signal by polymer layer 78.

### PRODUCING SENSING ELECTRODES INTENDED TO BE COUPLED TO DISTAL END ASSEMBLY

Fig. 4 is a block diagram that schematically illustrates a process flow for producing one of sensing electrodes 77 shown in Figs 2 and 3 above, in accordance with examples of the present disclosure.

At a step 1, polymer layer 76 is formed over the surface of gold substrate 75 such as by shrinking a polymer sleeve to provide a conformal surface. Note that the outer surface of polymer layer 76 is intended to be placed in contact with tissue, and polymer layer 76 is configured to electrically isolate between gold substrate 75 and the tissue in question.

At a step 2, a laser cutting system (not shown) is applied for cutting through polymer layer 76 and forming openings 79 at selected locations in which the second section of gold substrate 75 is exposed, as described for example in sectional view BB of Fig. 3 above. For the sake of conceptual clarity, polymer layer 76 includes sections 76a, 76b and 76c that are surrounding openings 79. Note that sections 76a, 76b and 76c of polymer layer 76 are all made from the same layer and have the same size and shape.

In some examples, sections 76a, 76b and 76c and openings 79 represent a portion of array 65 shown in Fig. 3 above. Thus, the sectional view of Fig. 4 typically continues in an XY plane of an XYZ coordinate system of Fig. 4, and typically includes additional sections of polymer layer 76 and additional openings 79.

In other examples, openings 79 may be formed using any other suitable patterning and/or material removal process.

At a step 3, polymer layer 78 is applied into openings 79 (e.g., between sections 76a and 76b, and between sections 76b and 76c of polymer layer 76), and are disposed over the exposed surface of gold substrate 75. Polymer layer 78 is electrochemically grown by placing the catheter into a coating liquid and then running current between electrodes on the catheter to a counter electrode placed in the coating fluid. Coating in this manner allows polymer layer 78 to selectively be grown only on desired surfaces.

In some examples, the outer surface of polymer layer 78 is intended to be placed in contact with the tissue in question, and polymer layer 78 is configured to conduct the ECG signals between the tissue in question and gold substrate 75.

Note that in accordance with the examples shown in Fig. 3 above, steps 1-3 of the process flow of Fig. 4 are designed to produce multiple sensing electrodes 77 in one batch on gold substrate 75. At a step 4 that concludes the process flow of Fig. 4, sensing electrodes 77 are produced by dicing polymer layer 76 and gold substrate 75, and subsequently, by separating between adjacent sensing electrodes 77. In the example of step 4, section 76b of polymer layer 76 is cut-through or diced, together with gold substrate 75, along the Z-axis of the XYZ coordinate system of Fig. 4.

In some examples, the process flow may comprise additional steps after dicing and separating one or more sensing electrodes 77. For example, such processes may comprise polishing and preparing one or more surfaces are required for coupling each sensing electrode 77 to a respective spline 55 of distal-end assembly 40.

In alternative examples, step 3 may be carried out after step 4 and after: (i) assembling sensing electrodes 77 and ablation electrodes 66 to respective splines 55, and (ii) applying a curing step to distal-end assembly 40 for outgassing polymers of distal-end assembly 40, and (iii) preparing the surface of gold substrate 75 of opening 79 for electrochemically growing polymer layer 78. Note that for the electrochemical coating of polymer layer 78 the surface of gold substrate 75 of opening 79 must be clean because residues, and particularly non-conductive residues, may interfere with the electrochemical growing of polymer layer 78. Moreover, the one or more curing step(s) of distal-end assembly 40 may pollute the surface of sensing electrode 77 with outgassing polymers that may reduce the electrical conductivity, and therefore, the sensitivity of sensing electrode 77.

### PRODUCING CATHETER HAVING ABLATION ELECTRODES THERMOCOUPLES AND SENSING ELECTRODES

Fig. 5 is a flow chart that schematically illustrates a method for producing catheter 22 and distal-end assembly 40 having ablation electrodes 66 and the sensing electrodes 77 shown in Figs. 2-4 above, in accordance with an example of the present disclosure.

The method begins at an electrically insulating layer (EIL) formation step 100, with applying polymer layer 76 to the surface of gold substrate 75, as described in detail in step 1 of Fig. 4 above.

At an EIL patterning step 102, a laser cutting system is used for removing sections of polymer layer 76, and producing openings 79 for exposing the surface of gold substrate 75, as described in detail in step 2 of Fig. 4 above.

At a dicing and separation step 104, section 76b of polymer layer 76 and gold substrate 75 are diced or otherwise being cut along the Z-axis, and each sensing electrode 77 is produced, as described in detail in step 3 of Fig. 4 above.

At a catheter assembly step 106, sensing electrodes 77 and ablation electrodes 66 are coupled to splines 55. Moreover, splines 55 are assembled for producing distal-end assembly 40, and subsequently, (i) distal-end assembly 40 is coupled to shaft 23, and (ii) at least distal-end assembly 40 (and optionally shaft 23) undergo one or more curing processes for outgassing polymers away from catheter 22.

At an electrically conducting (ECL) layer formation step 108 that concludes the method, polymer layer 78 is applied into openings 79 and is formed over the exposed surface of section gold substrate 75, as described in detail in step 3 of Fig. 4 above. Note that the outer surface of polymer layer 78 is typically flush with that of polymer layer 76, so as to enable a firm contact between the tissue in question and polymer layer 78, and thereby, conduct the ECG signal(s) between the tissue and gold substrate 75.

Note that the electrochemical coating of polymer layer 78 is carried out on a completed device (e.g., distal-end assembly 40 or catheter 22), so as to ensure that the coating of polymer layer 78 is not polluted with outgassing polymers during the curing steps of distal-end assembly 40 and/or catheter 22.

The method of Fig. 5 is simplified for the sake of conceptual clarity, and the production method typically comprises additional steps, such as but not limited to coupling position sensor 39 to distal-end assembly 40 and the aforementioned curing of catheter 22 (the curing is carried out using any suitable process, such as but not limited to an ultraviolet (UV) curing process or a thermal curing process. In the UV curing process, part of, or all of the device is exposed to UV light (which may be monochromatic or broad spectrum, but typically includes a wavelength of about 405 nm or below) in order to cure adhesives. In the thermal curing process part of, or all of the device is placed within an oven (typically at a temperature between about 40°C and 95°C, but most commonly at a temperature of about 65°C) to cure the adhesives. Moreover, various types of connectors are coupled to the proximal end of catheter 22, e.g., for exchanging the sensing signals and ablation signals between the components of console 24 and distal-end assembly 40.

Fig. 6 is a flow chart that schematically illustrates a method for producing catheter 22 having ablation electrodes 66 and one or more TCs 68, in accordance with an example of the present disclosure.

The method begins at an ablation electrode production step 200, with producing ablation electrodes 66, at least one of and typically all ablation electrodes 66 have TH 70 for threading splines therethrough, and slot 67 for containing TC 68 or any other temperature sensor, as described in detail in Fig. 2 above.

At a temperature sensor coupling step 202, TC 68 is coupled to slot 67 of ablation electrode 66, as described in detail in Fig. 2 above.

At a catheter assembly step 204 that concludes the method, ablation electrodes 66 are coupled to splines 55, as described in detail in Fig. 2 above. Moreover, splines 55 are assembled for producing distal-end assembly 40, and subsequently, distal-end assembly 40 is coupled to shaft 23 for concluding the production of catheter 22.

Note that the electrochemical coating of polymer layer 78, which is described in detail in Fig. 5 above, is typically carried out on a completed device (e.g., distal-end assembly 40 or catheter 22), so as to ensure that the coating of polymer layer 78 is not polluted with outgassing polymers during the curing steps of distal-end assembly 40 and/or catheter 22.

These particular flow charts of Figs. 5 and 6 are provided by way of example, in order to illustrate certain problems in sensing electro-anatomical signals in tissue in question, and in applying ablation signals to tissue intended to be ablated. These problems are addressed by examples of the present disclosure and are intended to demonstrate the application of these examples in enhancing the performance of the sensing and ablation system of Fig. 1 above. Examples of the present disclosure, however, are by no means limited to these specific sorts of methods and/or to this specific sort of example system, and the principles described herein may similarly be applied to other sorts of tissue sensing and/or ablating system.

In other examples, the methods of Figs. 5 and 6 may be merged into a single method for producing distal-end assembly 40 having a combination of suitable ablation electrodes (such as one or more ablation electrodes 66), temperature sensors (such as one or more TCs 68) and sensing electrodes (such as one or more sensing electrodes 77) that are all incorporated on distal-end assembly 40.

Although the examples described herein mainly address basket catheters, or other sort of expandable catheters, used for cardiac ablation. The methods and systems described herein can also be used in other applications, such as in neurology, otolaryngology, and renal denervation.

It will thus be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof which would occur to persons skilled in the art upon reading the foregoing description and which are not disclosed in the prior art. The invention is defined in the appended claims.

## Claims

1. A catheter (22), comprising:
a shaft (23) for insertion into an organ (26) of a patient (28);
an expandable distal-end assembly (40), which is coupled to the shaft (23) and comprises multiple splines (55), wherein at least one of the splines (55) comprises a flexible substrate (56), which is configured to conform to tissue of the organ (26); and
at least a sensing electrode (77), which is configured: (a) to be coupled to the flexible substrate (56), and (b) when placed in contact with tissue of the organ (26), to produce a electrical signal indicative of an electrocardiogram, ECG, signal sensed in the tissue, the sensing electrode (77) comprising:
(i) a gold substrate (75), which is formed on the flexible substrate (56) and is configured to conduct the electrical signal,
(ii) a first polymer layer (76), which is formed over a first section (76a, 76b, 76c) of the gold substrate (75) and is configured to electrically isolate between the tissue and the gold substrate (75), and
(iii) a second polymer layer (78), which is formed over a second section (79) of the gold substrate (75), different from the first section (76a, 76b, 76c), and is configured to conduct the electrical signal between the tissue and the gold substrate (75);
wherein the first section and the second section have a common interface and do not overlap one another.

2. The catheter according to claim 1, wherein the second section is positioned within an opening in the first section.

3. The catheter according to claim 1, wherein the organ comprises a heart, and comprising one or more ablation electrodes, which are coupled to the flexible substrate of at least one of the splines, and wherein when placed in contact with the tissue, at least one of the ablation electrodes is configured to apply to the tissue one or more ablation signals for producing a lesion in the tissue.

4. The catheter according to claim 3, wherein the ablation electrodes are positioned on the spline at first positions, and the sensing electrode is positioned on the spline at a second position, different from the first positions.

5. The catheter according to any one of claims 1-4, wherein at least one of the splines comprises one or more of the sensing electrodes that are coupled to the flexible substrate at selected positions along a longitudinal axis of the spline.

6. The catheter according to any one of claims 1-4, wherein the first and second polymer layers have first and second outer surfaces, respectively, which are intended to be placed in contact with the tissue, and wherein the second outer surface is recessed relative to the first outer surface.

7. The catheter according to any one of claims 1-4, wherein the second polymer layer includes a circular shape, and wherein the first polymer layer is shaped for surrounding the circular shape of the second polymer layer.

8. The catheter according to any one of claims 1-4, wherein the flexible substrate comprises an alloy of nickel-titanium, and wherein at least the sensing electrode is coupled to the alloy of nickel-titanium.

9. A method for producing a catheter (22) according to claim 1, the method comprising:
producing at least a sensing electrode (77) for sensing an electrocardiogram (ECG) signal in tissue of an organ (26) by:
forming a first polymer layer (76) over a first section (76a, 76b, 76c) of a gold substrate (75) for electrically isolating between the gold substrate (75) and the tissue; and
forming, over a second section (79) of the gold substrate (75), different from the first section(76a, 76b, 76c), a second polymer layer (78) for electrically conducting the ECG signal between the tissue and the gold substrate (75);
coupling the sensing electrode (77) to a flexible substrate (56) of a spline (55) of an expandable distal-end assembly (40), wherein when placed in contact with the organ (26), the spline (55) conforms to the tissue; and
coupling the expandable distal-end assembly (40) to a shaft (23) for insertion into the organ (23);
wherein the first section and the second section have a common interface and do not overlap one another.

10. The method according to claim 9, wherein forming the first polymer layer comprises applying the first polymer layer to the first and second sections, and exposing the second section of the gold substrate by removing the first polymer from the second section.

11. The method according to claim 10, wherein forming the second polymer layer comprises applying the second polymer layer to the exposed second section.

12. The method according to any one of claims 9-11, wherein the organ comprises a heart, and comprising coupling, to the flexible substrate of at least one of the splines, one or more ablation electrodes, such that when placed
in contact with the tissue, at least one of the ablation electrodes is used for applying to the tissue one or more ablation signals for producing a lesion in the tissue.

13. The method according to claim 12, wherein coupling the one or more ablation electrodes comprises positioning the one or more ablation electrodes on the spline at first positions, and positioning the sensing electrode on the spline at a second position, different from the first positions.

14. The method according to any one of claims 9-11, wherein the first and second polymer layers have first and second outer surfaces, respectively, which are intended to be placed in contact with the tissue, and wherein forming the first polymer layer and the second polymer layer comprises forming the second outer surface recessed relative to the first outer surface.

15. The method according to any one of claims 9-11, wherein the second polymer layer includes a circular shape, and wherein forming the first polymer layer comprises shaping the first polymer layer for surrounding the circular shape of the second polymer layer.

16. The catheter according to any one of claims 1-4 or the method according to any one of claims 9-11, wherein the first polymer layer comprises polyethylene terephthalate, PET, or polyether block amide, PEBA, and wherein the second polymer layer comprises poly(3,4-ethylenedioxythiophene), PEDOT, or poly(3, 4 ethylenedioxythiophene) polystyrene sulfonate, PEDOT:PSS.

17. The method according to any one of claims 9-11, wherein the flexible substrate comprises an alloy of nickel-titanium, and wherein coupling the sensing electrode comprises coupling at least the sensing electrode to the alloy of nickel-titanium.

## Patentansprüche

1. Katheter (22), umfassend:
einen Schaft (23) für ein Einführen in ein Organ (26) eines Patienten (28);
eine expandierbare Distalendanordnung (40), die mit dem Schaft (23) gekoppelt ist und mehrere Keile (55) umfasst, wobei mindestens einer der Keile (55) ein flexibles Substrat (56) umfasst, das konfiguriert ist, um sich an ein Gewebe des Organs (26) anzupassen; und
mindestens eine Erfassungselektrode (77), die konfiguriert ist: (a) um mit dem flexiblen Substrat (56) gekoppelt zu werden, und (b), wenn sie in Kontakt mit dem Gewebe des Organs (26) platziert wird, um ein elektrisches Signal zu erzeugen, das ein Elektrokardiogrammsignal, EKG-Signal, anzeigt, das in dem Gewebe erfasst wird, die Erfassungselektrode (77) umfassend:
(i) ein Goldsubstrat (75), das auf dem flexiblen Substrat (56) ausgebildet ist und konfiguriert ist, um das elektrische Signal zu leiten,
(ii) eine erste Polymerschicht (76), die über einem ersten Abschnitt (76a, 76b, 76c) des Goldsubstrats (75) ausgebildet ist und konfiguriert ist, um zwischen dem Gewebe und dem Goldsubstrat (75) elektrisch zu isolieren, und
(iii) eine zweite Polymerschicht (78), die über einem zweiten Abschnitt (79) des Goldsubstrats (75), der sich von dem ersten Abschnitt (76a, 76b, 76c) unterscheidet, ausgebildet ist und konfiguriert ist, um das elektrische Signal zwischen dem Gewebe und dem Goldsubstrat (75) zu leiten;
wobei der erste Abschnitt und der zweite Abschnitt eine gemeinsame Grenzfläche aufweisen und einander nicht überlappen.

2. Katheter nach Anspruch 1, wobei der zweite Abschnitt innerhalb einer Öffnung in dem ersten Abschnitt positioniert ist.

3. Katheter nach Anspruch 1, wobei das Organ ein Herz umfasst, und umfassend eine oder mehrere Ablationselektroden, die mit dem flexiblen Substrat von mindestens einem der Keile gekoppelt sind, und wobei, wenn sie in Kontakt mit dem Gewebe platziert werden, mindestens eine der Ablationselektroden konfiguriert ist, um ein oder mehrere Ablationssignale zum Erzeugen einer Läsion in dem Gewebe an das Gewebe anzulegen.

4. Katheter nach Anspruch 3, wobei die Ablationselektroden auf dem Keil an ersten Positionen positioniert sind und die Erfassungselektrode auf dem Keil an zweiten Positionen, die sich von den ersten Positionen unterscheiden, positioniert ist.

5. Katheter nach einem der Ansprüche 1 bis 4, wobei mindestens einer der Keile eine oder mehrere der Erfassungselektroden umfasst, die an ausgewählten Positionen entlang einer Längsachse des Keils mit dem flexiblen Substrat gekoppelt sind.

6. Katheter nach einem der Ansprüche 1 bis 4, wobei die erste und die zweite Polymerschicht eine erste beziehungsweise eine zweite Außenoberfläche aufweisen, die dazu bestimmt sind, in Kontakt mit dem Gewebe platziert zu werden, und wobei die zweite Außenoberfläche relativ zu der ersten Außenoberfläche zurückgesetzt ist.

7. Katheter nach einem der Ansprüche 1 bis 4, wobei die zweite Polymerschicht eine kreisförmige Gestalt einschließt, und wobei die erste Polymerschicht zum Umschließen der kreisförmigen Gestalt der zweiten Polymerschicht gestaltet ist.

8. Katheter nach einem der Ansprüche 1 bis 4, wobei das flexible Substrat eine Legierung aus Nickeltitan umfasst, und wobei mindestens die Erfassungselektrode mit der Legierung aus Nickeltitan gekoppelt ist.

9. Verfahren zum Erzeugen eines Katheters (22) nach Anspruch 1, das Verfahren umfassend:
Erzeugen mindestens einer Erfassungselektrode (77) zum Erfassen eines Elektrokardiogrammsignals (EKG-Signals) in dem Gewebe eines Organs (26) durch:
Ausbilden einer ersten Polymerschicht (76) über einem ersten Abschnitt (76a, 76b, 76c) eines Goldsubstrats (75) zum elektrischen Isolieren zwischen dem Goldsubstrat (75) und dem Gewebe; und
Ausbilden, über einem zweiten Abschnitt (79) des Goldsubstrats (75), der sich von dem ersten Abschnitt (76a, 76b, 76c) unterscheidet, einer zweiten Polymerschicht (78) zum elektrischen Leiten des EKG-Signals zwischen dem Gewebe und dem Goldsubstrat (75);
Koppeln der Erfassungselektrode (77) mit einem flexiblen Substrat (56) eines Keils (55) einer expandierbaren Distalendanordnung (40), wobei sich der Keil (55), wenn er in Kontakt mit dem Organ (26) platziert wird, an das Gewebe anpasst; und
Koppeln der expandierbaren Distalendanordnung (40) mit einem Schaft (23) für das Einführen in das Organ (23);
wobei der erste Abschnitt und der zweite Abschnitt eine gemeinsame Grenzfläche aufweisen und einander nicht überlappen.

10. Verfahren nach Anspruch 9, wobei das Ausbilden der ersten Polymerschicht ein Aufbringen der ersten Polymerschicht auf den ersten und den zweiten Abschnitt und ein Freilegen des zweiten Abschnitts des Goldsubstrats durch Entfernen des ersten Polymers von dem zweiten Abschnitt umfasst.

11. Verfahren nach Anspruch 10, wobei das Ausbilden der zweiten Polymerschicht das Aufbringen der zweiten Polymerschicht auf den freigelegten zweiten Abschnitt umfasst.

12. Verfahren nach einem der Ansprüche 9 bis 11, wobei das Organ ein Herz umfasst, und umfassend das Koppeln, mit dem flexiblen Substrat von mindestens einem der Keile, von einer oder mehrerer Ablationselektroden, sodass, wenn sie in Kontakt mit dem Gewebe platziert werden, mindestens eine der Ablationselektroden zum Anlegen eines oder mehrerer Ablationssignalen an das Gewebe zum Erzeugen einer Läsion in dem Gewebe verwendet wird.

13. Verfahren nach Anspruch 12, wobei das Koppeln der einen oder der mehreren Ablationselektroden ein Positionieren der einen oder der mehreren Ablationselektroden auf dem Keil an ersten Positionen und das Positionieren der Erfassungselektrode auf dem Keil an einer zweiten Position, die sich von den ersten Positionen unterscheidet, umfasst.

14. Verfahren nach einem der Ansprüche 9 bis 11, wobei die erste und die zweite Polymerschicht die erste beziehungsweise die zweite Außenoberfläche aufweisen, die dazu bestimmt sind, in Kontakt mit dem Gewebe platziert zu werden, und wobei das Ausbilden der ersten Polymerschicht und der zweiten Polymerschicht das Ausbilden der zweiten Außenoberfläche zurückgesetzt relativ zu der ersten Außenoberfläche umfasst.

15. Verfahren nach einem der Ansprüche 9 bis 11, wobei die zweite Polymerschicht eine kreisförmige Gestalt einschließt, und wobei das Ausbilden der ersten Polymerschicht das Ausbilden der ersten Polymerschicht zum Umschließen der kreisförmigen Gestalt der zweiten Polymerschicht umfasst.

16. Katheter nach einem der Ansprüche 1 bis 4 oder Verfahren nach einem der Ansprüche 9 bis 11, wobei die erste Polymerschicht Polyethylenterephthalat, PET, oder Polyetherblockamid, PEBA, umfasst, und wobei die zweite Polymerschicht Poly(3,4-ethylendioxythiophen), PEDOT, oder Poly(3,4-ethylendioxythiophen)polystyrolsulfonat, PEDOT:PSS, umfasst.

17. Verfahren nach einem der Ansprüche 9 bis 11, wobei das flexible Substrat eine Legierung aus Nickeltitan umfasst, und wobei das Koppeln der Erfassungselektrode das Koppeln von mindestens der Erfassungselektrode mit der Legierung aus Nickeltitan umfasst.

## Revendications

1. Cathéter (22), comprenant :
une tige (23) pour une insertion dans un organe (26) d'un patient (28) ;
un ensemble d'extrémité distale (40) extensible, qui est accouplé à la tige (23) et comprend plusieurs languettes (55), dans lequel au moins l'une des languettes (55) comprend un substrat flexible (56), qui est conçu pour épouser la forme du tissu de l'organe (26) ; et
au moins une électrode de détection (77), qui est configurée : (a) pour être accouplée au substrat flexible (56), et (b) lorsqu'elle est placée en contact avec le tissu de l'organe (26), pour produire un signal électrique indiquant un signal d'électrocardiogramme, ECG, détecté dans le tissu, l'électrode de détection (77) comprenant :
(i) un substrat en or (75), qui est formé sur le substrat flexible (56) et est configuré pour conduire le signal électrique,
(ii) une première couche polymère (76), qui est formée sur une première section (76a, 76b, 76c) du substrat en or (75) et est configurée pour créer une isolation électrique entre le tissu et le substrat en or (75), et
(iii) une seconde couche polymère (78), qui est formée sur une seconde section (79) du substrat en or (75), différente de la première section (76a, 76b, 76c), et est configurée pour conduire le signal électrique entre le tissu et le substrat en or (75) ;
dans lequel la première section et la seconde section ont une interface commune et ne se chevauchent pas.

2. Cathéter selon la revendication 1, dans lequel la seconde section est positionnée à l'intérieur d'une ouverture dans la première section.

3. Cathéter selon la revendication 1, dans lequel l'organe comprend un coeur, et comprenant une ou plusieurs électrodes d'ablation, qui sont accouplées au substrat flexible d'au moins l'une des languettes, et dans lequel, lorsqu'elle est placée en contact avec le tissu, au moins l'une des électrodes d'ablation est configurée pour appliquer au tissu un ou plusieurs signaux d'ablation pour la production d'une lésion dans le tissu.

4. Cathéter selon la revendication 3, dans lequel les électrodes d'ablation sont positionnées sur la languette à des premières positions, et l'électrode de détection est positionnée sur la languette à une seconde position, différente des premières positions.

5. Cathéter selon l'une quelconque des revendications 1 à 4, dans lequel au moins l'une des languettes comprend une ou plusieurs des électrodes de détection qui sont accouplées au substrat flexible à des positions sélectionnées le long d'un axe longitudinal de la languette.

6. Cathéter selon l'une quelconque des revendications 1 à 4, dans lequel les première et seconde couches polymères ont des première et seconde surfaces externes, respectivement, qui sont destinées à être placées en contact avec le tissu, et dans lequel la seconde surface externe est en retrait par rapport à la première surface externe.

7. Cathéter selon l'une quelconque des revendications 1 à 4, dans lequel la seconde couche polymère comporte une forme circulaire, et dans lequel la première couche polymère est mise en forme pour entourer la forme circulaire de la seconde couche polymère.

8. Cathéter selon l'une quelconque des revendications 1 à 4, dans lequel le substrat flexible comprend un alliage nickel-titane, et dans lequel au moins l'électrode de détection est accouplée à l'alliage nickel-titane.

9. Procédé permettant de produire un cathéter (22) selon la revendication 1, le procédé comprenant :
la production d'au moins une électrode de détection (77) pour la détection d'un signal d'électrocardiogramme (ECG) dans le tissu d'un organe (26) par :
la formation d'une première couche polymère (76) sur une première section (76a, 76b, 76c) d'un substrat en or (75) pour créer une isolation électrique entre le substrat en or (75) et le tissu ; et
la formation, sur une seconde section (79) du substrat en or (75), différente de la première section (76a, 76b, 76c), d'une seconde couche polymère (78) pour la conduction électrique du signal ECG entre le tissu et le substrat en or (75) ;
l'accouplement de l'électrode de détection (77) à un substrat flexible (56) d'une languette (55) d'un ensemble d'extrémité distale (40) expansible, dans lequel, lorsqu'elle est placée en contact avec l'organe (26), la languette (55) épouse la forme du tissu ; et
l'accouplement de l'ensemble d'extrémité distale (40) expansible à une tige (23) pour une insertion dans l'organe (23) ;
dans lequel la première section et la seconde section ont une interface commune et ne se chevauchent pas.

10. Procédé selon la revendication 9, dans lequel la formation de la première couche polymère comprend l'application de la première couche polymère sur les première et seconde sections, et l'exposition de la seconde section du substrat en or en retirant le premier polymère de la seconde section.

11. Procédé selon la revendication 10, dans lequel la formation de la seconde couche polymère comprend l'application de la seconde couche polymère sur la seconde section exposée.

12. Procédé selon l'une quelconque des revendications 9 à 11, dans lequel l'organe comprend un cœur, et comprenant l'accouplement, au substrat flexible d'au moins l'une des languettes, d'une ou de plusieurs électrodes d'ablation, de telle sorte que, lorsqu'elle est placée en contact avec le tissu, au moins l'une des électrodes d'ablation est utilisée pour appliquer au tissu un ou plusieurs signaux d'ablation pour la production d'une lésion dans le tissu.

13. Procédé selon la revendication 12, dans lequel l'accouplement de la ou des électrodes d'ablation comprend le positionnement de la ou des électrodes d'ablation sur la languette à des premières positions, et le positionnement de l'électrode de détection sur la languette à une seconde position, différente des premières positions.

14. Procédé selon l'une quelconque des revendications 9 à 11, dans lequel les première et seconde couches polymères ont des première et seconde surfaces externes, respectivement, qui sont prévues pour être placées en contact avec le tissu, et dans lequel la formation de la première couche polymère et de la seconde couche polymère comprend la formation de la seconde surface externe en retrait par rapport à la première surface externe.

15. Procédé selon l'une quelconque des revendications 9 à 11, dans lequel la seconde couche polymère comporte une forme circulaire, et dans lequel la formation de la première couche polymère comprend la mise en forme de la première couche polymère pour entourer la forme circulaire de la seconde couche polymère.

16. Cathéter selon l'une quelconque des revendications 1 à 4 ou procédé selon l'une quelconque des revendications 9 à 11, dans lequel la première couche polymère comprend du polyéthylène téréphtalate, PET, ou du polyéther bloc amide, PEBA, et dans lequel la seconde couche polymère comprend du poly(3,4-éthylènedioxythiophène), PEDOT, ou du poly(3,4-éthylènedioxythiophène)polystyrène sulfonate, PEDOT : PSS.

17. Procédé selon l'une quelconque des revendications 9 à 11, dans lequel le substrat flexible comprend un alliage nickel-titane, et dans lequel l'accouplement de l'électrode de détection comprend l'accouplement au moins de l'électrode de détection à l'alliage nickel-titane.
